# EUROPEAN PATENT SPECIFICATION

(11) **EP 3 664 687 B1**
(45) Date of publication and mention of the grant of the patent: **15.05.2024**
(21) Application number: 18843004.5
(22) Date of filing: 09.08.2018
(51) Int. Cl.: A61B 1/24, A61B 5/00, A61B 1/06, A61B 1/04, A61C 9/00

(54) **INTRA-ORAL SCANNING DEVICE**
INTRAORALE SCANVORRICHTUNG
DISPOSITIF DE BALAYAGE INTRAORAL

(30) Priority: 10.08.2017 US 201762543557 P
(43) Date of publication of application: 17.06.2020
(73) Proprietor: D4D Technologies, LLC, Richardson, TX 75081 (US)
(72) Inventor: LI, Ye, Richardson TX 75081 (US); QUADLING, Mark, S., Richardson TX 75081 (US); QUADLING, Henley, S., Richardson TX 75081 (US); DUNCAN, Rod, A., Richardson TX 75081 (US); BASILE, Gregory, R., Richardson TX 75081 (US); GRAHAM, Justin, G., Richardson TX 75081 (US); KENWORTHY, Grant, E., Richardson TX 75081 (US); TCHOUPRAKOV, Andrei, Richardson TX 75081 (US); TOIMELA, Lasse, H., Richardson TX 75081 (US)
(74) Representative: TLIP Limited
(86) International application number: PCT/US2018/046144
(87) International publication number: WO 2019/032923

(56) References cited:
- EP-A1- 0 345 368
- KR-A- 20110 127 950
- KR-A- 20150 111 122
- KR-B1- 101 717 284
- US-A1- 2002 045 811
- US-A1- 2010 253 773
- US-A1- 2013 130 191
- US-B2- 9 539 070

## Description

### BACKGROUND OF THE INVENTION

### Technical Field

This disclosure relates generally to scanning devices.

### Brief Description of the Related Art

It is known to provide an intra-oral scanner to enable a user to scan dental patients intra-orally. Such devices are used in a standalone scanner, or as part of a computer-aided design and manufacture (CAD/CAM) system. A CAD/CAM system typically uses dental CAD software executing on a laptop or desktop machine, optionally together with specialized milling machine hardware driven by machine control CAM software. The dentist first prepares a patient's damaged tooth anatomy (using standardized dental practices) to receive a dental restoration including, but not limited to, an inlay, an onlay, a veneer, a crown or a bridge. Once the preparation has been made, the dentist uses the scanner described and illustrated herein to capture a digital impression of a patient's dental anatomy. Once the digital impression has been captured the dentist is presented with an "initial proposal" restoration by the automated CAD software. This initial proposal preferably automatically selects an appropriate tooth anatomy, and it sizes it to fit onto the preparation and within the patient's existing "good" anatomy. This initial proposal is then customized by the dental professional, typically using specialized software tools to adjust and modify the design, with the goal of ultimately achieving an optimized design that fits into the patient's anatomy. Once the final 3D model of the tooth has been achieved, it is sent electronically to a milling machine (or third party), which then generates the actual restoration from the design.

While existing scanner devices provide satisfactory results, there remains a need for improvements in scanning speed and accuracy, as well as to reduce the size and weight of the device to thereby make it easier to use in practice.
KR-10-2015-0111122 relates to three-dimensional scanners, and more particularly, to three-dimensional scanners that can be suitably used for acquiring stereoscopic image data in a narrow space such as the oral cavity or a narrow internal space.

### BRIEF SUMMARY

An intra-oral scanning device is provided to more efficiently and accurately scan dental patients intra-orally. The device typically comprises a component of an optical impression system for computer-aided design (CAD) and manufacture (CAM) of dental restorations. In operation, the device is used for recording topological characteristics of teeth, dental impressions, or stone models by digital methods and for use in CAD/CAM of dental restorative prosthetic devices. According to this disclosure, various operating components in the device are configured and arranged so as to simplify the mechanical and electrical packaging and assembly, and accordingly the scanner is much more compact and easier to use as compared to prior art intra-oral scanners.

The foregoing has outlined some of the more pertinent features of the subject matter. These features should be construed to be merely illustrative.
An intra-oral scanner is the first aspect of the present invention and is provided in claim 1. Preferred embodiments are provided in the dependent claims. Any embodiments of the disclosure below which are not encompassed by the claims are provided for reference only.

### BRIEF DESCRIPTION OF THE DRAWINGS

For a more complete understanding of the disclosed subject matter and the advantages thereof, reference is now made to the following descriptions taken in conjunction with the accompanying drawings, in which:
FIG. 1 depicts a perspective view of an embodiment of a hand-held scanner according to this disclosure;
FIG. 2 depicts a light engine module of the scanner shown in a perspective view;
FIG. 2A depicts a cutaway (interior) view of the light engine module;
FIG. 3 depicts a despeckler module of the scanner in a perspective view;
FIG. 3A depicts an interior view of the despeckler module;
FIG. 4 depicts a light projection module of the scanner in a perspective view;
FIG. 4A depicts an interior view of the light projection module;
FIG. 5 depicts a lens tube module of the scanner in a perspective view;
FIG. 5A depicts an interview of the lens tube module;
FIG. 6 depicts a preferred construction of the TIR prism in the light engine module of the scanner;
FIG. 7A depicts the beam path through the light engine module for light that is directed at greater than normal and thus projected to the rest of the optical system;
FIG. 7B depicts the beam path through the light engine module for light that is directed at less than normal and thus not projected;
FIG. 8A is a plan view of the optical system of the scanner;
FIG. 8B is an elevation view of the optical system;
FIG. 9 depicts a component-specific view of a preferred embodiment of the scanner; and
FIG. 10 depicts a twist lock mechanism for attaching the scanner tip to the scanner body.

### DETAILED DESCRIPTION

As noted above, the scanner of this disclosure is a handheld optical scanner that is designed to be placed in a patient's mouth to create an image (typically a 3D image) of the teeth after preparation for dental restoration. The following describes an embodiment of this scanner.

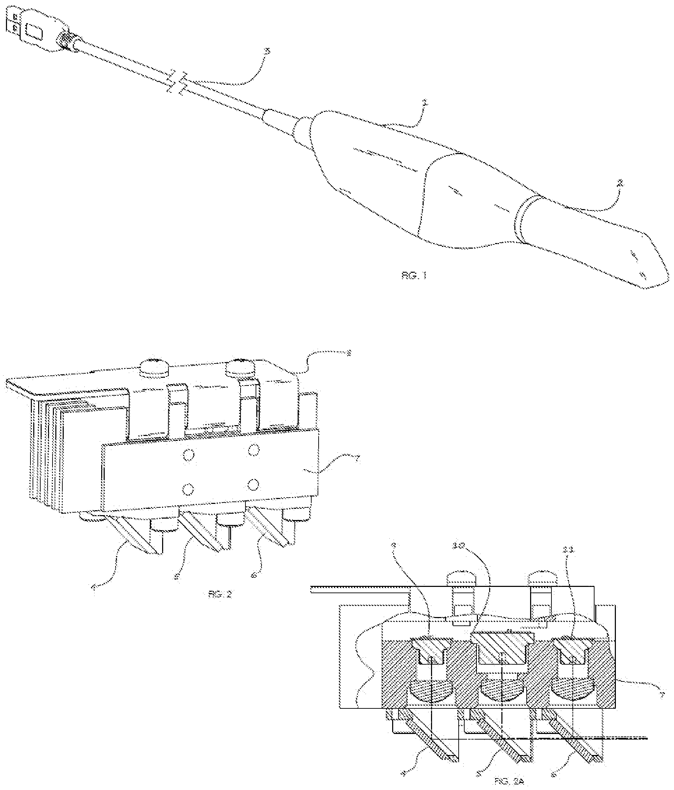

In particular, FIG. 1 depicts a perspective view of the hand-held scanner in one embodiment. In this embodiment, the scanner preferably comprises a scanner body 1, a detachable scanner tip 2, and detachable data cable 3.

With reference now to FIG. 2, a light engine module of the scanner of FIG. 1 is shown in perspective, and FIG. 2A provides an interior (cutaway) view of the light engine module. The light engine module preferably comprises red laser diode 9, green laser diode 10, and blue laser diode 11. A full spectrum mirror 4, a red passing and green reflecting dichroic filter 5, and blue reflecting, red and green passing dichroic filter 6, respectively, are positioned adjacent the diodes. Element 7 is a laser housing and heat sink for the module, and element 8 is a laser flexible circuit board to which the laser diodes are mounted.

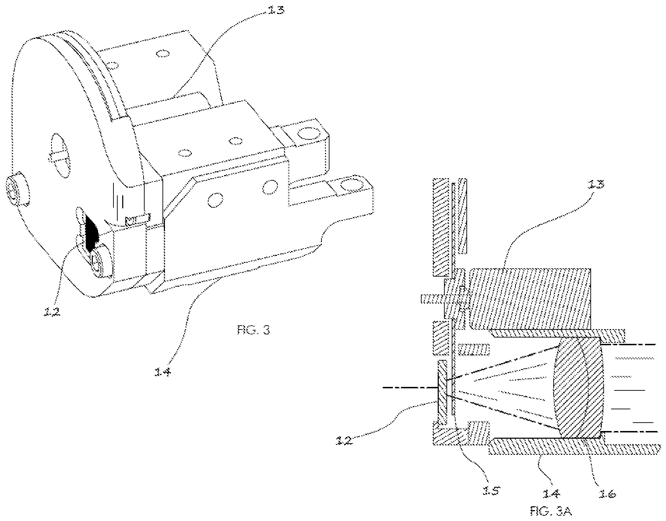

FIG. 3 depicts a despeckler module, and FIG. 3A provides an interior view of the despeckler module. The despeckler module comprises micro lens array (MLA) 12, a despeckler drive motor 13, a despeckler housing 14, a diffuser disk 15 (that acts as a despeckling element), and an achromatic lens 16 (a "doublet" or "collimating" lens). The diffuser disk spins in front of the laser. In an alternative embodiment, the diffuser may move by other means in the vertical, horizontal, circular, or random axes.

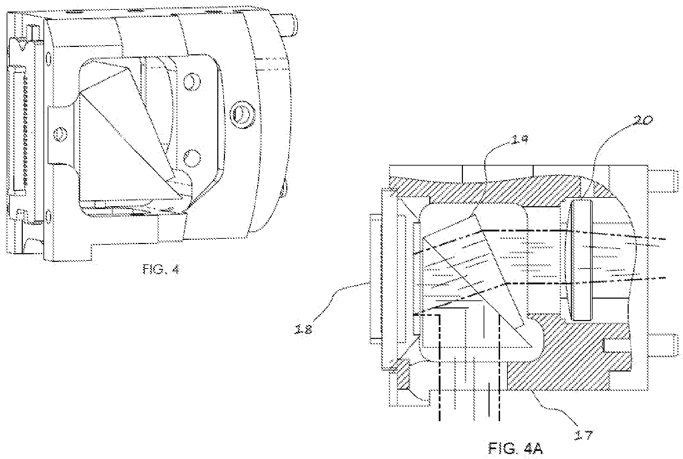

FIG. 4 depicts a projection module in perspective, and FIG. 4A depicts a cutaway view showing the light paths. As shown, the projection module comprises a TIR housing 17, a laser light spatial modulator chip 18 (Texas Instruments DLP^{®}), a Total Internal Reflection (TIR) prism 19, and a tele-centric lens 20. As depicted, the light comes into the module normal to the modulator chip surface; that light is then moved off-axis by the TIR prism 19. As will be described in more detail below, this configuration enables the size of the overall optics system to be substantially reduced, thereby enabling the overall scanner to be reduced in size.

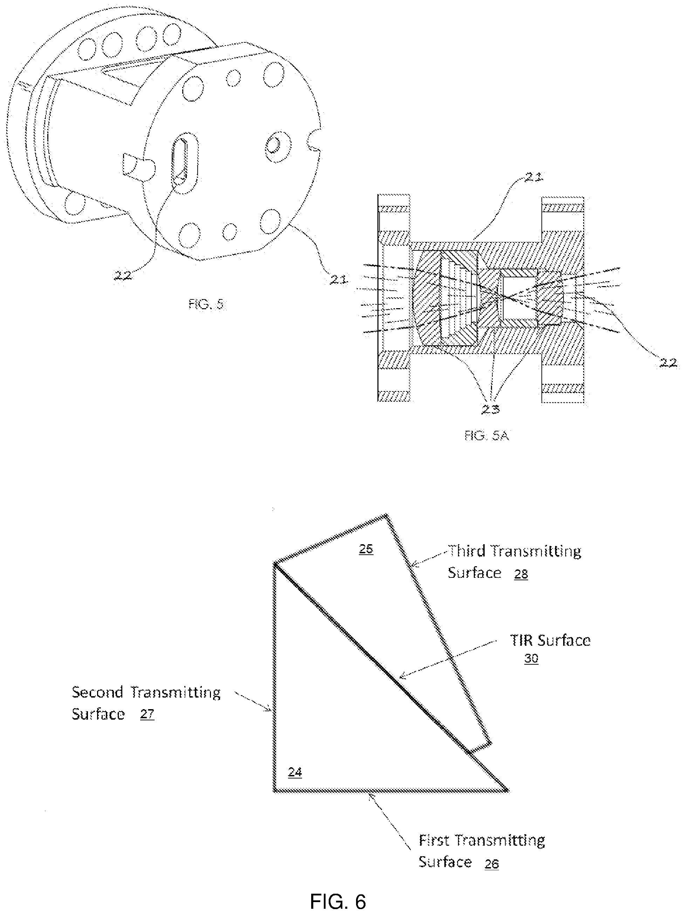

FIG. 5 depicts a lens tube module in perspective, and FIG. 5A depicts a cutaway view. As best seen in the perspective view, the module includes a magnification lens housing 21 (lens barrel) that includes a slotted (sometimes referred to herein as a "cat-eye") aperture 22. Magnification lenses 23 are supported substantially as shown. The aperture 22 provides significantly enhanced depth-of-field for the laser lines that comprise the projected image. In effect, and by using the aperture, the optics system sacrifices resolution in the vertical direction while significantly enhancing resolution in the horizontal direction. The notion here is to provide more optical power in the direction that matters to the imaging process.

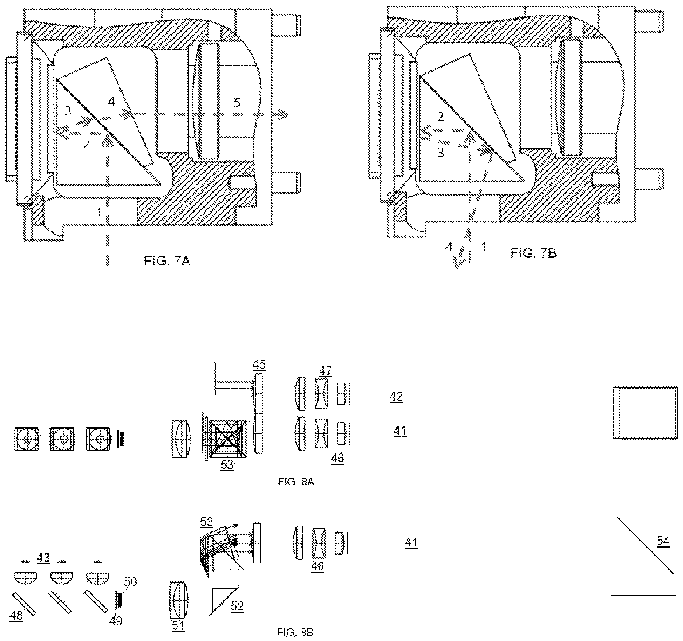

The following provides additional details regarding the Total Internal Reflection (TIR) prism shown in FIG. 4, as well as its principle of operation. As depicted in FIG. 6, the TIR prism preferably is comprised of two pieces of glass, numbered 24 and 25, which pieces preferably are glued together with a very small air gap between them. The TIR prism is configured to transmit light that comes into the prism at a certain range of angles, and to reflect light that comes in at a different angle. This operation can be seen in FIG. 6 with respect to three (3) identified transmitting surfaces, as well as the TIR surface itself 30. As noted above, the TIR prism is used to transmit light of a certain incident angle and reflect light of a different incident angle. Light enters the prism at a normal to a first transmitting surface 26 and is largely reflected off of the TIR surface 30. This light is transmitted out of a second transmitting surface 27 onto a DMD surface 28, which will direct the light either at an angle greater than normal or less than normal depending in the DMD micro-mirrors' position. The light then again transits through the second transmitting surface 27. Light that exits the DMD surface 28 at an angle greater than normal is transmitted through the TIR surface 30 and is then transmitted out of the prism through a third transmitting surface 29. This is depicted in FIG. 7A. The third transmitting surface 29 is angled such that the exiting light is transmitted normal to the remaining projection path. Light that is reflected at an angle less than normal reflects off of the TIR surface 30 and is not projected through the system. This is depicted in FIG. 7B.

Thus, the TIR prism preferably is comprised of two prisms that are configured as shown, preferably with a few micron air gap there-between. The first prism 24 is a triangle (or right angle) prism comprising one angle at 90° and two other equal angles (at 45°), and it is formed of a material selected to ensure total internal reflection at surface 30. The second prism 25 is also a triangle prism, and it is formed in a shape of a wedge prism in which the wedge angle and material are designed to make the exiting laser beam parallel to the optical axis. Other than the 90° angle, the second prism has angles of approximately 21 and 69 degrees. As noted, preferably the prisms are bonded together with a small air gap along the surface 30. Preferably, the prisms are sized to ensure that there is a sufficient optically-clear aperture to cover the pattern size of laser beam. As noted above, the laser beam enters the first prism 24 at normal incident angle, and it is internally reflected (totally) by the 45° TIR surface 30 such that the beam then hits on the light modulator. When the modulator is turned on, and when each individual mirror turns +12 degree, then the laser beam is reflected back to prism 24 through to the 45° TIR surface 30. Due to the DMD angle, there is no internal reflection at the surface 30 of prism 24. Thus, the laser beam travels through the first prism and reaches the second prism 25, where it is then bent by the third transmitting (back) surface such that the laser beam is parallel to the optical axis and goes through to the rest of the optical path. As noted above, this operation is depicted in FIG. 7A. When the DMD is at a parked position of 0° degree or at OFF position of angle of -12° degree, the laser beam does not make it through the 45° surface 30 of the first prism due to total internal reflection.

The above-described manner of arranging the TIR configuration enables both the DMD chip and the CCD (or CMOS-based) chip to be positioned in a vertical plane, and it simplifies the mechanical and electrical packaging and assembly. In part due to this construction, the overall scanner is much more compact than prior devices of this type.

In an alternative embodiment, the relative positions of the two prisms are switched, in which case the exit laser beam is normal to the TIR surface of the 45° prism, and the DMD chip is in a horizontal plane and perpendicular to CCD (or CMOS) surface.

FIGS. 8A and 8B depict the scanner's optical system in additional detail. FIG. 8A is a plan view, and FIG. 8B is an elevation view. As best depicted in FIG. 8A, the scanner's optical system 40 is configured to include two (2) optical paths, namely, a laser projection path 41, and an optical imaging path 42. Generally, the laser projection path preferably comprises three (3) color (RGB) lasers 43, and a spatial light modulator 44 (e.g., Texas Instruments DLP^{®} light modulator) to project a structured laser light pattern and live view color illumination on the tooth surface. The optical imaging path 42 comprises a high speed and high-resolution CCD (or CMOS) sensor 45 to capture the image of the laser light pattern projected on the tooth surface from a perspective view. The separation of the two optical paths (which are configured side-by-side as depicted) forms a triangulation between a projected laser light pattern and the CCD optical imaging such a 3D shape of the tooth surface can be determined based on well-known triangulation principles. Preferably, both the projecting lenses 46 and the imaging lenses 47 each include the same four lens group and are optimized for high resolution, color correction, and tele-centric rays in the imaging space. In addition to the three (3) color laser diodes 43, the laser projection path includes laser collimating lenses 48, color combining filters 49, a micro-lens array homogenizer 50, a laser speckle reducer 51, an achromatic doublet lens 52, and a reflective TIR (Total Internal Reflection) prism 53 (as previously described). At the end of the scanner tip, the transmitted light is reflected off mirror 54.

Preferably, the depth of the field (approximately 15 mm) in the optical imaging path is designed based on controlling of aperture stop size and focal length. The depth of the field (e.g., approximately 15 mm) in the laser projection path is designed based on a slit aperture stop (as will be described in more detail below) to achieve sharp laser lines and bright laser output. The field of view (e.g., approximately 17 mm x 13 mm) is designed based on the selected CCD sensor and spatial light modulator size, tip mirror size, optical magnification and total optical length. Preferably, a small imaging aperture stop and projection aperture stop located at the front of the optical system and without using any glass window, and preferably all of the lenses are attached to the main mechanical housing to avoid fogging in the optical path with the tip mirror, which is preferably heated.

Without intended to be limiting, representative optical design parameters of the scanner are as follows: effective focal length (26.6 mm), triangulation angle (6.55°), magnification (1/3.6^{x}), field of view (17.6 mm x 13.2 mm), CCD sensor size (4.736 x 3.552 mm with 7.4µ m pixel, 200 fps), spatial light modulator (0.3" with 10.6µm pitch in column), color (3 lasers with RGB color), contrast (on and off mirror switching), uniformity (flat - top illumination with micro lens array).

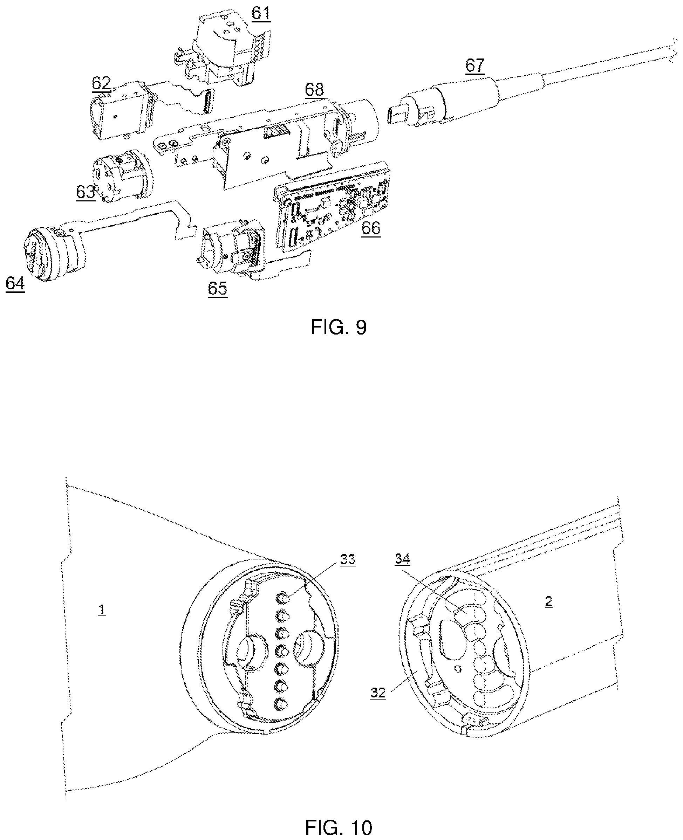

Referring now to FIG. 9, a component-specific view of a preferred embodiment of the scanner is shown in additional detail. The plastic case that houses these components is not shown. As depicted, in this embodiment the scanner 60 comprises despeckler module 61, projection module 62, lens tube module 63 (with the cat-eye slotted aperture), tip mount module 64, camera module 65, electronics module 66, data cable 67, and light engine module 68.

The cat-eye aperture of the lens tube module provides additional advantages. In operation, and as depicted in FIG. 8A, the light exiting from the TIR prism goes through the lens tube module (that supports the four lens projection system 46). The lens tube module includes the cat-eye (or "stop") aperture having a slotted shape. Advantageously, the slot is configured along the laser line direction, thereby allowing more laser power to go through the system. The narrow direction of the aperture produces sufficient depth of field for the thin and sharp laser lines. Preferably, the lens projection system 46 is identical to the adjacent imaging system 47, which is optimized for high resolution and high depth of field for 3D measurement. Typically, the imaging system has a stop aperture of circular shape. The four lens system is a tele-centric design in imaging space for improved transmission and detection.

Preferably, and with reference again to FIG. 1, the scanner tip 2 and data cable 3 are detachable and are replaceable components. The data cable 3 that attaches the scanner to a computer is a USB 3.0 data cable preferably attached to the remainder of the device by a bayonet lock style connector.

In operation, scanning software resident on an associated computer (e.g., desktop, laptop, or the like) extracts a 3D point cloud from the captured data, aligns the 3D point cloud to previously captured data, and renders to a display screen (or other output). This process is repeated as the user continues to scan. The system then allows the user to bring the restored anatomical data into a design tool. Through the use of the software, the user then designs a restoration (e.g., a crown) to fit the anatomical features.

Preferably, the scanner tip's mechanical design is a one-piece plastic housing, preferably with no external seams. It may also include an orientation marking to facilitate use. A mirror in the tip preferably is heated to prevent fogging, which would otherwise negatively impact the clinical experience. As depicted in FIG. 10, the tip 2 is attached to the scanner body 1 using a twist lock mechanism 32. By rotating the body relative to the tip, the tip can be removed for service or replacement. Electrical connectivity to the heated mirror is provided by a connector structure, which includes contacts 33 on the body, a contact pad 34 comprising a set of pogo pad contacts. The electrical connectivity provided by the contacts 33 includes power, communications (e.g., in one specific case I²C), and safety.

Preferably, the RGB lasers in the scanner are color-balanced to produce a desirable image as is now described. In particular, the approach herein uses color calibration via laser emitter balancing. The following describes an approach to this calibration process.

Each laser has a specified frequency range (i.e. red, green or blue), and the pulse width or power of each emitter is adjustable. As used herein, an "emitter" refers to the LEDs or lasers that illuminate the scene, "emitter driver value" refers to the value (e.g., pulse width or other electrical power) that drives the apparent amplitude of the emitter, and a value "tRGB" is a desired or target mean RGB value of a calibration target. To carry out the calibration process, the wand is first placed on a color calibration target that is greyscale. A target RGB value for the resulting image is then set to tRGB. An emitter driver value in the middle of an allowed range (that is configurable) is then selected. A snapshot of the target is then taken and the mean RGB values collected. A determination is then made whether the mean RGB value is greater than tRGB, and the result is used as an initial condition for a binary search. The emitter driver values are then adjusted using a binary search until a delta between the mean RGB and tRGB is minimized. The resulting optimized emitter driver values are then used to drive the color frames of the scanner (i.e., during normal use). Preferably, tRGB is selected such that green and blue have much stronger components than red, as this reduces the amount of red scattering in the patient's mouth. In an alternative embodiment, in lieu of greyscale, different color spaces (e.g., HSL, HSV) may be used to drive the calibration.

According to another aspect, color uniformity correction may be carried out as follows. The scanner is first placed on a color calibration target that is greyscale. The scene is then illuminated, preferably based on the optimized emitter driver values as described above. The frame is then captured. Then, the frame is blurred, e.g., using an n x n kernel. For each pixel, a scale factor is the calculated. The scale factor is a value that maps an input RGB to a desired output RGB that is similar to rRGB. The scale factor image is then compressed (e.g., using OpenJPEG), which reduces grid compression artifacts while significantly reducing file size. This compressed file is then stored to the scanner. Upon the start of scanning, this scale factor image is multiplied by the incoming scanner image to correct uniformity errors. The scale factor image is calculated and used in a pair of equations, the first equation being S = T/I, derived during calibration (and assuming element-by-element arithmetic operations), where S is the scale factor image, I is the incoming image from the scanner, and T is the image with tRGB at every pixel; the second equation being O = S*I, which represents the output after calibration (i.e., during scanning), where S is the scale factor image, I is the incoming image from the scanner, and O is the output image displayed to the user.

According to a further aspect, the following describes an efficient way to reduce shadows due to laser emitters residing on a different path from the image sensor. In this aspect, a kd-tree is computed from the generated 3D model. For each vertex on the generated model, and using the kd-tree, a ray is cast from the vertex to an estimated camera position. The intersected result is then stored. The routine preferably uses an epsilon along the ray to assure that the ray is not intersecting a test vertex. Using the kd-tree, a ray also is cast from the vertex to the estimated laser illumination position, and the intersected result also is stored. An epsilon also is used along the ray to assure the ray is not intersecting the test vertex. The color from the live view image is looked up only if the camera ray and laser ray are not occluded by other geometry.

Typically, the frames used to capture the data for the 3D model are partially-illuminated frames. To facilitate the operation of the device and provide live video as feedback to the operator (as well as the 3D-computed data), typically the scanner uses a sequence of patterns throughout which full illumination frames are selectively interspersed. A full illumination frame involves all or substantially all lines being turned on, as compared to a partially-illuminated approach, wherein only some lines are projected. In a full illumination frame, in effect there is no pattern. The partially-illustrated frames provide the data from which the 3D coordinates of the surface are determined. A technique for rendering frames in this manner is described in U.S. Patent No. 7,184,150. In contrast, the full illumination frames are used for texturing the 3D model generated by the partially-illuminated frame data. In one sequence, a first set (e.g., six) pattern frames are used, interspersed with a second set (e.g., three) illumination frames, for a sequence total of nine total CCD frames. A software traffic shaper is then used to separate captured frames in two streams, namely, a live preview stream, and a data processing stream from which the 3D model is generated. If necessary, e.g., for computational or storage efficiencies, the live preview stream can give up priority and drop some frames when the CPU work load exceeds a certain limit.

As noted above, the intraoral scanner described herein may be provided as a standalone scanner, or as part of a CAD/CAM system. In one non-limiting implementation, the scanner is part of a CAD/CAM system that uses dental CAD software, such as E4D Design Center, executing on a laptop or desktop machine, optionally together with specialized milling machine hardware driven by machine control CAM software. The dentist first prepares a patient's damaged tooth anatomy (using standardized dental practices) to receive a dental restoration including, but not limited to, an inlay, an onlay, a veneer, a crown or a bridge. Once the preparation has been made, the dentist uses the scanner described and illustrated herein to capture a digital impression of a patient's dental anatomy. Once the digital impression has been captured the dentist is presented with an "initial proposal" restoration by the automated CAD software. This initial proposal preferably automatically selects an appropriate tooth anatomy, and it sizes it to fit onto the preparation and within the patient's existing "good" anatomy. This initial proposal is then customized by the dental professional, typically using specialized software tools to adjust and modify the design, with the goal of ultimately achieving an optimized design that fits into the patient's anatomy. Once the final 3D model of the tooth has been achieved, it is sent electronically to a milling machine (or third party), which then generates the actual restoration from the design.

The RGB lasers in the scanner may be selectively controlled (or turned off) to produce any particular color (e.g., blue, purple, etc.). In another embodiment, the particular color utilized for scanning is a function of the material to be scanned.

The scanner tip also may be customized as needed (e.g., to include additional devices or elements) depending on the scanning application. The electrical interface to the tip provides greater customization possibilities by providing power, communication, and safety to the tip designer.

## Claims

1. An intra-oral scanner, comprising:
a body (1);
a tip (2) attached to the body (1);
a light engine module; and
a projection module, the projection module comprising a light modulator, and first (24) and second (25) prism elements, the first prism element (24) being a right angle- shaped prism having a TIR surface (30), and the second prism element (25) being a wedge-shaped prism positioned adjacent the TIR surface (30), the first prism element (24) configured to receive light generated by the light engine module, and to reflect the received light off the light modulator;
wherein light that exits a surface of the light modulator at an angle greater than normal is transmitted through the first (24) and second (25) prism elements out of the TIR prism, and light that exits the surface of the light modulator at an angle less than normal is reflected by the first element (24) and not transmitted through the second element (25).

2. The intra-oral scanner as described in claim 1 wherein the light engine module comprises a red (R) laser diode (9), a green (G) laser diode (10), and a blue (B) laser diode (11).

3. The intra-oral scanner as described in claim 1 further including a laser projection subsystem.

4. The intra-oral scanner as described in claim 3 wherein the laser projection subsystem comprises a housing (7) that supports one or more projection lens that receive light that exits the TIR prism.

5. The intra-oral scanner as described in claim 4 wherein a surface of the housing includes an elongated aperture, the aperture elongated along a direction of a set of lines of a light pattern to be transmitted from the scanner.

6. The intra-oral scanner as described in claim 1 further including a despeckler module downstream of the light engine module, optionally wherein the despeckler module comprises a rotating diffuser disk, or optionally wherein the despeckler module includes a micro lens array (12) that is configured as a light homogenizer to make laser patterns generated by the light engine more uniform.

7. The intra-oral scanner as described in claim 1 wherein the tip (2) is seamless.

8. The intra-oral scanner as described in claim 2 further including an electronics module that provides color calibration of laser diodes in the light engine module, optionally wherein color calibration captures an image from a greyscale target, sets a target RGB value for the image to a target mean RGB value, selects emitter driver values for the laser diodes within a configurable range, collects mean RGB values, and thereafter adjusts the emitter driver values until a difference between the mean RGB and target mean RGB is minimized.

9. The intra-oral scanner as described in claim 1 wherein the scanner body (1) supports a laser projection sub-system and an optics imaging sub-system side-by-side to reduce a form factor of the scanner body (1).

10. The intra-oral scanner as described in claim 1 further including a laser projection sub- system that includes a tele-centric lens (20).

11. The intra-oral scanner as described in claim 2 further including a full spectrum mirror (4), a red passing and green reflecting dichroic filter (5), and a blue reflecting, red and green passing dichroic filter (6) adjacent the laser diodes.

12. The intra-oral scanner as described in claim 1 wherein the tip (2) includes electrical connectivity to provide power to a heated mirror supported in the tip (2).

## Patentansprüche

1. Ein Intraoralscanner, der Folgendes beinhaltet:
einen Körper (1);
eine Spitze (2), die an dem Körper (1) befestigt ist;
ein Lichtmaschinenmodul; und
ein Projektionsmodul, wobei das Projektionsmodul einen Lichtmodulator und ein erstes (24) und ein zweites (25) Prismenelement beinhaltet, wobei das erste Prismenelement (24) ein rechtwinklig geformtes Prisma mit einer TIR-Oberfläche (30) ist und das zweite Prismenelement (25) ein keilförmiges Prisma ist, das angrenzend an die TIR-Oberfläche (30) positioniert ist, wobei das erste Prismenelement (24) so konfiguriert ist, dass es von dem Lichtmaschinenmodul erzeugtes Licht empfängt und das empfangene Licht von dem Lichtmodulator reflektiert;
wobei Licht, das aus einer Oberfläche des Lichtmodulators in einem Winkel, der größer als normal ist, austritt, durch das erste (24) und das zweite (25) Prismenelement aus dem TIR-Prisma heraus weitergeleitet wird und Licht, das aus der Oberfläche des Lichtmodulators in einem Winkel, der kleiner als normal ist, austritt, von dem ersten Element (24) reflektiert und nicht durch das zweite Element (25) weitergeleitet wird.

2. Intraoralscanner gemäß Anspruch 1, wobei das Lichtmaschinenmodul eine rote (R) Laserdiode (9), eine grüne (G) Laserdiode (10) und eine blaue (B) Laserdiode (11) beinhaltet.

3. Intraoralscanner gemäß Anspruch 1, der ferner ein Laserprojektions-Subsystem umfasst.

4. Intraoralscanner gemäß Anspruch 3, wobei das Laserprojektions-Subsystem ein Gehäuse (7) beinhaltet, das eine oder mehrere Projektionslinsen trägt, die Licht empfangen, das aus dem TIR-Prisma austritt.

5. Intraoralscanner gemäß Anspruch 4, wobei eine Oberfläche des Gehäuses eine langgestreckte Öffnung aufweist, wobei die Öffnung entlang einer Richtung eines Satzes von Linien eines von dem Scanner weiterzuleitenden Lichtmusters langgestreckt ist.

6. Intraoralscanner gemäß Anspruch 1, der ferner ein dem Lichtmodul nachgeschaltetes Despecklermodul beinhaltet, wobei das Despecklermodul optional eine rotierende Diffusorscheibe beinhaltet oder wobei das Despecklermodul optional ein Mikrolinsenarray (12) beinhaltet, das als Lichthomogenisator konfiguriert ist, um die von der Lichtmaschine erzeugten Lasermuster einheitlicher zu machen.

7. Intraoralscanner gemäß Anspruch 1, wobei die Spitze (2) nahtlos ist.

8. Intraoralscanner gemäß Anspruch 2, der ferner ein Elektronikmodul umfasst, das eine Farbkalibrierung von Laserdioden in dem Lichtmaschinenmodul bereitstellt, wobei die Farbkalibrierung optional ein Bild von einem Graustufenziel erfasst, einen Ziel-RGB-Wert für das Bild auf einen mittleren Ziel-RGB-Wert festsetzt, Emittertreiberwerte für die Laserdioden innerhalb eines konfigurierbaren Bereichs auswählt, mittlere RGB-Werte sammelt und anschließend die Emittertreiberwerte einstellt, bis eine Differenz zwischen dem mittleren RGB und dem mittleren Ziel-RGB minimiert ist.

9. Intraoralscanner gemäß Anspruch 1, wobei der Scannerkörper (1) ein Laserprojektions-Subsystem und ein optisches Abbildungs-Subsystem nebeneinander trägt, um den Formfaktor des Scannerkörpers (1) zu reduzieren.

10. Intraoralscanner gemäß Anspruch 1, der ferner ein Laserprojektions-Subsystem umfasst, das eine telezentrische Linse (20) umfasst.

11. Intraoralscanner gemäß Anspruch 2, der ferner einen Vollspektrumspiegel (4), einen für Rot durchlässigen und Grün reflektierenden dichroitischen Filter (5) und einen Blau reflektierenden, für Rot und Grün durchlässigen dichroitischen Filter (6) neben den Laserdioden umfasst.

12. Intraoralscanner gemäß Anspruch 1, wobei die Spitze (2) eine elektrische Verbindungsfähigkeit aufweist, um einem in der Spitze (2) getragenen beheizten Spiegel Leistung bereitzustellen.

## Revendications

1. Un dispositif de balayage intra-buccal, comprenant :
un corps (1) ;
un embout (2) fixé au corps (1) ;
un module générateur de lumière ; et
un module de projection, le module de projection comprenant un modulateur de lumière, et des premier (24) et deuxième (25) éléments prismes, le premier élément prisme (24) étant un prisme conformé en angle droit ayant une surface TIR *(Total Internal Reflection,* réflexion totale interne) (30), et le deuxième élément prisme (25) étant un prisme conformé en coin positionné adjacent à la surface TIR (30), le premier élément prisme (24) étant configuré pour recevoir de la lumière générée par le module générateur de lumière, et pour réfléchir la lumière reçue, par le modulateur de lumière ;
où de la lumière qui sort d'une surface du modulateur de lumière à un angle supérieur à la normale est transmise à travers les premier (24) et deuxième (25) éléments prismes hors du prisme TIR, et de la lumière qui sort de la surface du modulateur de lumière à un angle inférieur à la normale est réfléchie par le premier élément (24) et n'est pas transmise à travers le deuxième élément (25).

2. Le dispositif de balayage intra-buccal tel que décrit dans la revendication 1 où le module générateur de lumière comprend une diode laser (9) rouge (R), une diode laser (10) verte (G), et une diode laser (11) bleue (B).

3. Le dispositif de balayage intra-buccal tel que décrit dans la revendication 1 incluant en outre un sous-système de projection laser.

4. Le dispositif de balayage intra-buccal tel que décrit dans la revendication 3 où le sous-système de projection laser comprend un boîtier (7) qui supporte une ou plusieurs lentilles de projection qui reçoivent de la lumière qui sort du prisme TIR.

5. Le dispositif de balayage intra-buccal tel que décrit dans la revendication 4 où une surface du boîtier inclut une ouverture allongée, l'ouverture étant allongée suivant une direction d'un ensemble de lignes d'un motif lumineux devant être transmis à partir du dispositif de balayage.

6. Le dispositif de balayage intra-buccal tel que décrit dans la revendication 1 incluant en outre un module déchatoyeur en aval du module générateur de lumière, facultativement où le module déchatoyeur comprend un disque diffuseur rotatif, ou facultativement où le module déchatoyeur inclut un réseau de microlentilles (12) qui est configuré comme un homogénéiseur de lumière afin de rendre des motifs laser générés par le générateur de lumière plus uniformes.

7. Le dispositif de balayage intra-buccal tel que décrit dans la revendication 1 où l'embout (2) est sans soudure.

8. Le dispositif de balayage intra-buccal tel que décrit dans la revendication 2 incluant en outre un module de dispositifs électroniques qui fournit un étalonnage de couleur de diodes laser dans le module générateur de lumière, facultativement où l'étalonnage de couleur capture une image à partir d'une cible à échelle de gris, règle une valeur RGB cible pour l'image sur une valeur RGB moyenne cible, sélectionne des valeurs de pilote émetteur pour les diodes laser au sein d'une plage configurable, recueille des valeurs RGB moyennes, et ensuite ajuste les valeurs de pilote émetteur jusqu'à ce qu'une différence entre la RGB moyenne et la RGB moyenne cible soit minimisée.

9. Le dispositif de balayage intra-buccal tel que décrit dans la revendication 1 où le corps (1) de dispositif de balayage supporte un sous-système de projection laser et un sous-système d'imagerie d'optique côte à côte afin de réduire un facteur de forme du corps (1) de dispositif de balayage.

10. Le dispositif de balayage intra-buccal tel que décrit dans la revendication 1 incluant en outre un sous-système de projection laser qui inclut une lentille télécentrique (20).

11. Le dispositif de balayage intra-buccal tel que décrit dans la revendication 2 incluant en outre un miroir à spectre complet (4), un filtre dichroïque (5) laissant passer le rouge et réfléchissant le vert, et un filtre dichroïque (6) réfléchissant le bleu, laissant passer le rouge et le vert, adjacents aux diodes laser.

12. Le dispositif de balayage intra-buccal tel que décrit dans la revendication 1 où l'embout (2) inclut un connecteur électrique afin de fournir une alimentation à un miroir chauffé supporté dans l'embout (2).
